# EUROPÄISCHE PATENTANMELDUNG

(11) **EP 0 761 820 A1**
(43) Veröffentlichungstag der Anmeldung: **12.03.1997**
(21) Anmeldenummer: 96113250.3
(22) Anmeldetag: 19.08.1996
(51) Int. Cl.: C12Q 1/28, C12Q 1/26, G01N 33/52

(54) **Nachweissystem und Verfahren für die qualitative und quantitative Bestimmung von Wasserstoffperoxid, Substraten, aus denen unter der Einwirkung von Oxidasen Wasserstoffperoxid gebildet wird, und Halogeniden**

(30) Priorität: 07.09.1995 DE 19533072
(71) Anmelder: FRAUNHOFER-GESELLSCHAFT ZUR FÖRDERUNG DER ANGEWANDTEN FORSCHUNG E.V., 80636 München (DE)
(72) Erfinder: Kühn, Manfred, Dr. Dipl.-Chem., 13125 Berlin (DE); Wiesner, Wolfgang, Dr. Dipl.-Bio., 70794 Filderstadt (DE)
(74) Vertreter: Weisert, Annekäte, Dipl.-Ing. Dr.-Ing.

(57) **Zusammenfassung**

Beschrieben wird ein Nachweissystem zur qualitativen und quantitativen Bestimmung von Wasserstoffperoxid, Substraten, aus denen unter der Einwirkung von Oxidasen Wasserstoffperoxid gebildet wird, und Halogeniden, das dadurch gekennzeichnet ist, daß i) zur Bestimmung von Wasserstoffperoxid das Nachweissystem (a) mindestens einen halogenierbaren Farbstoff, b) mindestens ein halogenierendes Enzym, und (c) mindestens ein anorganisches Halogenid enthält; oder ii) zur Bestimmung von Substraten, aus denen unter der Einwirkung von Oxidasen Wasserstoffperoxid gebildet wird, das Nachweissystem (a) mindestens einen halogenierbaren Farbstoff, (b) mindestens ein halogenierendes Enzym, (c) mindestens ein anorganisches Halogenid, und (d) mindestens eine Oxidase enthält; oder iii) zur Bestimmung von Halogenid das Nachweissystem (a) mindestens einen halogenierbaren Farbstoff, (b) mindestens ein halogenierendes Enzym, und (e) Wasserstoffperoxid enthält.

## Beschreibung

Die Erfindung betrifft ein Nachweissystem und ein Verfahren für die qualitative und quantitative Bestimmung von Wasserstoffperoxid, Substraten, aus denen unter der Einwirkung von Oxidasen Wasserstoffperoxid gebildet wird, und Halogeniden.

Durch enzymatische Oxidationsreaktionen kann eine Vielzahl von Verbindungen qualitativ und quantitativ sowohl mit Testlösungen als auch mit Teststreifen bestimmt werden. Besonders vorteilhaft gelingt das mit den zu den Oxidoreduktasen gehörenden Oxidasen. Bei diesen Enzymreaktionen oxidiert das Enzym aus der Gruppe der Oxidasen das Enzymsubstrat, wobei gleichzeitig von einem Akzeptor die aus der Oxidationsreaktion stammenden Elektronen aufgenommen werden. Für die Oxidasen ist der natürliche Elektronenakzeptor molekularer Sauerstoff, der mit zwei aus dem Oxidasesubstrat stammenden Protonen in der enzymkatalysierten Reaktion Wasserstoffperoxid bildet (Brockhaus ABC Biochemie, Brockhaus Verlag Leipzig, 1. Auflage, (1973), Seite 195; Römpps Chemie Lexikon, Franckh-sche Verlagsbuchhandlung, Stuttgart, 8. Auflage, 1985, Band 4, Seite 2946 und Seite 2952). Das entstandene Wasserstoffperoxid kann in Folgereaktionen Leukofarbstoffe durch Oxidation in Farbstoffe überführen, die wiederum durch Kupplungsreaktionen stabilisiert werden können. Solche oxidativen Kupplungsreaktionen sind bekannt (Angewandte Chemie, 70. 215, (1958)) und können auch durch Oxidoreduktasen, speziell durch Peroxidase oder auch andere peroxidatisch aktive Verbindungen, verwirklicht werden (DE-OS 33 31 588).

Mit bisher bekannten Nachweissystemen (vgl. US-PS 3 092 465, US-PS 3 802 842 und US-PS 3 867 258), wie z.B. bei handelsüblichen Papierteststreifen, die aus mit Indikatorlösungen getränkten Filterpapieren bestehen, läßt sich allenfalls eine halbquantitative Bestimmung von Wasserstoffperoxid, Substraten, aus denen unter der Einwirkung von Oxidasen Wasserstoffperoxid gebildet wird, erreichen. Diese Teststreifen enthalten in der Regel farblose und oxidierbare aromatische Verbindungen, sogenannte Leukofarbstoffe, die durch Wasserstoffperoxid und mit Hilfe des Enzyms Peroxidase oder einer anderen peroxidatisch aktiven Verbindung in mehr oder weniger stabile und farbige Produkte überführt werden und auf diese Weise einen optisch-visuellen Nachweis erlauben. Das zu bestimmende Wasserstoffperoxid kann aus einer rein chemischen, aber auch aus einer durch eine Enzymreaktion, in der Regel durch Oxidasen, vermittelten Reaktion herrühren. Da bei einer solchen Enzymreaktion das Wasserstoffperoxid aus dem Oxidasesubstrat und Sauerstoff gebildet wird, ist es möglich, über die Bestimmung des Wassersoffperoxids das Enzymsubstrat, das stöchiometrisch in die Enzymreaktion eingeht, nachzuweisen bzw. zumindest halbquantitativ zu bestimmen. Halogenide wurden bisher allerdings nach diesem enzymatisch-chemischen Meßprinzip, das auch als trockenchemisches Meßprinzip bezeichnet wird, nicht bestimmt.

Die bekannten Nachweissysteme besitzen jedoch erhebliche Nachteile. In vielen Fällen sind die verwendeten Leukofarbstoffe gegenüber Sauerstoff empfindlich, so daß erhebliche Probleme bei der Lagerstabilität der entwickelten Testlösungen oder Teststreifen entstehen können. Aber auch viele der zum Nachweis gebildeten Farbstoffe sind über längere Zeit nicht stabil. Das führt dazu, daß bei der Anwendung z.B. der Teststreifen ein vom Hersteller vorgegebener Zeitablauf zum Betrachten und Auswerten des Teststreifens nach Farbstoffbildung eingehalten werden muß, der in der Praxis durch den Anwender oft nicht eingehalten wird bzw. oft auch nur schwer einzuhalten ist. Bei diesen Papierteststreifen ist auch von Nachteil, daß die Ergebnisse der Auswertung, die mit Farbskalen (Komperatoren) erfolgt, durch unterschiedliche Lichtverhältnisse und dem variierenden Farbunterscheidungsvermögen des Anwenders beeinflußt werden. Daraus erklärt sich auch, daß mit solchen Papierteststreifen lediglich eine halbquantitative Bestimmung von Wasserstoffperoxid, Substraten, aus denen unter der Einwirkung von Oxidasen Wasserstoffperoxid gebildet wird, möglich ist und dabei nur unbefriedigende Ergebnisse erreicht werden.

Wesentlich verbessert wurde das Teststreifenprinzip zum trockenchemischen Nachweis von Wasserstoffperoxid, Substraten, aus denen unter der Einwirkung von Oxidasen Wasserstoffperoxid gebildet wird, durch die Entwicklung von transparenten Teststreifen, die z.B. in der US-PS 4 042 335, US-PS 4 050 898, EP-A-0 016 387, DE-A-32 47 608, EP-A-0 262 445 oder EP-A-0 256 806 beschrieben sind. Es beruht auf den Grundlagen der Bilderzeugung in der Farbfotografie. Diese Teststreifen besitzen eine wesentlich verbesserte Farbstabilität, und sie können mit Hilfe eines Spektralphotometers auch noch nach Stunden oder Tagen auch quantitativ ausgewertet werden. Diese Teststreifensysteme einschließlich neu entwickelter Meßprinzipien, Klein- und Großphotometer haben seit einigen Jahren Eingang in ärztliche Praxen und Großlaboratorien der klinischen Diagnostik gefunden, inzwischen aber auch nach Vereinfachung der Meßprinzipien und Meßgeräte im patientennahen Bereich, z.B. in Patientenhaushalten. Mit diesen transparenten Teststreifen können eine Vielzahl nieder- und hochmolekularer Verbindungen, z.B. Enzyme und ihre Aktivitäten, nachgewiesen und quantitativ bestimmt werden. Entsprechend ihrer enorm gewachsenen Bedeutung liegt über Teststreifensysteme zur Bestimmung von Wasserstoffperoxid eine umfangreiche Literatur vor, unter anderem in Form von Übersichtsartikeln und Monografien [Clin. Chem. 24, 1335 (1978); Trockenchemie, Analytik mit trägergebundenen Reagenzien, Georg Thieme Verlag, Stuttgart-New York, (1988)]. Der Halogenidnachweis auf enzymatisch-chemischer Grundlage wurde aber auch mit transparenten Teststreifen bisher nicht entwickelt bzw. beschrieben.

Bei den genannten transparenten Teststreifen zur Bestimmung von Wasserstoffperoxid, Substraten, aus denen unter der Einwirkung von Oxidasen Wasserstoffperoxid gebildet wird, erfolgt die Stabilisierung der primären gebildeten Farbstoffe über die Kupplung mit geeigneten, aus der Farbfotografie bekannten Farbkupplern (vgl. Moderne photographische Systeme, Verlag Grundstoffindustrie, Leipzig (1983)). Die in diesen Teststreifen verwirklichte oxidative Farbentwicklung und Farbkupplung wird in der Regel auch hier durch Oxidasen und das Enzym Peroxidase oder andere peroxidatisch aktive Verbindungen katalysiert. Als peroxidatisch aktive Verbindungen für die Anwendung in Teststreifen, allerdings mit geringerer Effektivität, können deshalb auch zur Peroxidase strukturverwandte Verbindungen eingesetzt werden. Als Hämine, Häminkomplexe, Hämoglobin und Cytochrome sind diese nieder- und hochmolekularen Verbindungen mit Pseudoperoxidaseaktivität dem Fachmann bekannt (Brockhaus ABC Biochemie, Brockhaus Verlag Leipzig, 1. Auflage (1973), Seite 423). Die beschriebenen Nachweissysteme in Form transparenter Teststreifen zur Bestimmung von Wasserstoffperoxid und Substraten, aus denen durch Einwirkung einer Oxidase Wasserstoffperoxid gebildet wird, betreffen Teststreifen, in deren Indikatorschicht, die auf der transparenten Folie fixiert ist, deshalb oxidierbare Leukofarbstoffkomponenten, z.B. aromatische Verbindungen, wie Diamine und Aminophenole, und C-H-acide Verbindungen, wie Phenole, Naphthole, Acetessigesterderivate, Pyrazolone und ähnliche, als Farbkuppler zusammen mit einer Oxidase und/oder einer peroxidatisch aktiven Verbindung in einem quellfähigen, transparenten Gel, gegebenenfalls zusammen mit einem Weichmacher und/oder einer oberflächenaktiven Verbindung (Netzmittel), vorliegen.

Ein besonderer Nachteil dieser transparenten Teststreifen ist die Anwesenheit hochtoxischer und erwiesenermaßen cancerogen wirkender Indikatorchemikalien (Leukofarbstoffe, Kupplungsreagentien) in der Indikatorschicht, die aufgrund ihrer Wasserlöslichkeit sowohl für den Anwender als auch für die Umwelt gefährlich sind. Weiterhin nachteilig ist bei diesen Teststreifensystemen die Verwendung der peroxidatisch aktiven Verbindungen, wie z.B. von Peroxidase, den Cytochromen, Hämoglobin, Häminen und Häminkomplexen, da sie gegenüber dem zu bestimmenden Wasserstoffperoxid empfindlich sind und leicht inaktiviert werden.

Der Erfindung liegt deshalb die Aufgabe zugrunde, Nachweissysteme in Form von Testlösungen und analytischen Elementen (Teststreifen) für den qualitativen Nachweis und die quantitative Bestimmung von Wasserstoffperoxid, Substraten, aus denen unter der Einwirkung von Oxidasen Wasserstoffperoxid gebildet wird, und Halogeniden zu entwickeln, die keine toxischen bzw. cancerogenen Leukofarbstoffe und Kupplungsreagentien enthalten, und in denen die Bildung der stabilen Farbstoffe aus den nichttoxischen und nichtcancerogenen Leukofarbstoffen durch eine gegenüber Wasserstoffperoxid stabilere Verbindung katalysiert wird. Die Stabilität der Farbstoffe über längere Zeit, die Empfindlichkeit, der Meßbereich und die Handhabung sollen so gestaltet werden, daß eine zeitliche Trennung von analytischem Test und Auswertung möglich wird, ein breiter Bereich linearer Abhängigkeit zwischen der Konzentration des Wasserstoffperoxids und der Farbkonzentration (Extinktion) erreicht wird und daß die Anwendung, z.B. des Teststreifens, auch durch ungeschultes Personal erfolgen kann.

Diese Aufgabe wird durch die Bereitstellung eines Nachweissystems für die qualitative und quantitative Bestimmung von Wasserstoffperoxid, Substraten, aus denen unter der Einwirkung von Oxidasen Wasserstoffperoxid gebildet wird, und Halogeniden gelöst, das i) zur Bestimmung von Wasserstoffperoxid das Nachweissystem (a) mindestens einen halogenierbaren Farbstoff, (b) mindestens ein halogenierendes Enzym, und (c) mindestens ein anorganisches Halogenid enthält; oder ii) zur Bestimmung von Substraten, aus denen unter der Einwirkung von Oxidasen Wasserstoffperoxid gebildet wird, das Nachweissystem (a) mindestens einen halogenierbaren Farbstoff, (b) mindestens ein halogenierendes Enzym, (c) mindestens ein anorganisches Halogenid, und (d) mindestens eine Oxidase enthält; oder iii) zur Bestimmung von Halogenid das Nachweissystem (a) mindestens einen halogenierbaren Farbstoff, (b) mindestens ein halogenierendes Enzym, und (e) Wasserstoffperoxid enthält.

Überraschend wurde gefunden, daß die Halogenierung von nichttoxischen und nichtcancerogenen, auch in der Lebensmittelindustrie eingesetzten Farbstoffen durch Halogenasen, vorzugsweise hämfreie Halogenasen, unter Verbrauch von Wasserstoffperoxid und einem Halogenid zu diffusionsfesten und über die Zeit stabilen Farbstoffen mit im Vergleich zu den Ausgangsfarbstoffen veränderten Farben führt, so daß ein für die praktische Anwendung geeigneter Nachweis und/oder eine Bestimmung von Wasserstoffperoxid, Substraten, aus denen unter der Einwirkung von Oxidasen Wasserstoffperoxid gebildet wird, und Halogeniden möglich ist, der eine zeitliche Trennung von analytischem Test und Auswertung erlaubt. Dabei sind die Farbänderungen infolge hoher Farbausbeuten und Intensitäten und guter Unterscheidbarkeit auch durch ungeschultes Personal gut erkennbar und erlauben neben einer qualitativen auch eine halbquantitative und quantitative Auswertung.

Der in dem erfindungsgemäßen Nachweissystem verwendete halogenierbare Farbstoff kann jeder beliebige Farbstoff sein, solange er den folgenden Maßgaben genügt:
i) er darf nicht toxisch bzw. cancerogen sein,
ii) er muß in Gegenwart eines halogenierenden Enzyms, eines anorganischen Halogenids und von Wasserstoffperoxid halogenierbar sein,
iii) er muß bei der Halogenierung seine Farbe ändern.

Farbstoffe, die diesen Maßgaben genügen, sind beispielsweise Triarylmethanfarbstoffe, bevorzugt Phenolrot, Chlorphenolrot, Kresolrot, Kresolpurpur, Thymolblau, Xylenolblau und Xylenolorange.

Die in dem erfindungsgemäßen Nachweissystem verwendeten halogenierenden und dabei Wasserstoffperoxid und Halogenide verbrauchenden Enzyme sind Halogenasen, vorzugsweise hämfreie Halogenasen, z.B. solche aus Pseudomonas pyrrocinia und Streptomyces aureofaciens. Diese hämfreien Halogenasen zeichnen sich durch eine erhöhte Stabilität gegenüber Wasserstoffperoxid aus, so daß auch die Möglichkeit zum Nachweis und zur Bestimmung hoher Konzentrationen von Wasserstoffperoxid besteht.

Das in dem erfindungsgemäßen Nachweissystem verwendete bzw. bestimmte anorganische Halogenid kann jedes beliebige anorganische Halogenid sein. Beispiele für solche Halogenide sind Alkali- und Erdalkalichloride, -bromide und -jodide. Besonders bevorzugt ist die Verwendung von Alkali- und Erdalkalibromiden.

Sehr vorteilhaft ist die Verwendung des erfindungsgemäßen Nachweissystems für die Bestimmung von Oxidasesubstraten allein oder in Stoffgemischen, z.B. zur Bestimmung von Glucose, Galactose, Lactose, Raffinose, Stachyose, Melibiose, Lactat, Cholin, Cholesterin, Harnsäure, L- oder D-Aminosäuren, Methanol, Ethanol, Xanthin, Aminen und/oder anderen Substraten. Besonders die in der klinischen Diagnostik des Diabetes mellitus oder der Galactosämie wichtigen Parameter Glucose bzw. Galactose, Lactose, Raffinose, Stachyose und Melibiose können vorteilhaft mit dem erfindungsgemäßen Nachweissystem bestimmt werden.

Dieses enthält dann je nach Art des zu analysierenden Substrats die entsprechende Oxidase, wie z.B. Glucoseoxidase, Galactoseoxidase, Lactatoxidase, Cholinoxidase, Cholesterinoxidase, Uricase, L- bzw. D-Aminosäureoxidase, Alkoholoxidase, Xanthinoxidase, Monoaminoxidase und/oder eine andere Oxidase bei der Bestimmung eines der hier nicht genannten Substrate, die die entsprechenden Substrate unter Bildung von Wasserstoffperoxid oxidieren.

Die Mengenverhältnisse der Komponenten (a) bis (e) in dem erfindungsgemäßen Nachweissystem unterliegen keinen besonderen Beschränkungen.

Zweckmäßigerweise liegen die Mengenverhältnisse der Komponenten (a), (b) und (c) bei der Bestimmung von Wasserstoffperoxid pro cm² Indikatorfläche des Teststreifensystems bzw. 5 ml Testlösung für (a) im Bereich von 0,1 mg bis 20 mg, vorzugsweise von 1 mg bis 10 mg, für (b) im Bereich von 0,5 Aktivitätseinheiten der Halogenase bis 50 Aktivitätseinheiten der Halogenase, vorzugsweise 5 bis 20 Aktivitätseinheiten, und für (c) im Bereich von 0,01 g bis 2 g, vorzugsweise 0,5 g bis 2 g.

Die Mengenverhältnisse der Komponenten (a), (b), (c) und (d) liegen bei der Bestimmung von Substraten, aus denen unter der Einwirkung von Oxidasen Wasserstoffperoxid gebildet wird, pro cm² Indikatorfläche des Teststreifensystems bzw. 5 ml Testlösung für (a) im Bereich von 0,1 mg bis 20 mg, vorzugsweise von 1 mg bis 10 mg, für (b) im Bereich von 0,5 Aktivitätseinheiten der Halogenase bis 50 Aktivitätseinheiten der Halogenase, vorzugsweise 5 bis 20 Aktivitätseinheiten, für (c) im Bereich von 0,01 g bis 2 g, vorzugsweise 0,5 g bis 2 g, und für (d) im Bereich von 0,5 Aktivitätseinheiten der Oxidase bis 30 Aktivitätseinheiten der Oxidase, vorzugsweise 3 bis 15 Aktivitätseinheiten.

Die Mengenverhältnisse der Komponenten (a), (b) und (e) liegen bei der Bestimmung von Halogeniden pro 5 ml Testlösung für (a) im Bereich von 0,1 mg bis 20 mg, vorzugsweise von 1 mg bis 10 mg, für (b) im Bereich von 0,5 Aktivitätseinheiten der Halogenase bis 50 Aktivitätseinheiten der Halogenase, vorzugsweise 5 bis 20 Aktivitätseinheiten, und für (e) im Bereich von 0,1 mMol bis 15 mMol, vorzugsweise von 1 mMol bis 10 mMol.

Wie bereits oben ausgeführt, kann das erfindungsgemäße Nachweissystem in Form einer Lösung oder als analytisches Element im Sinne eines Teststreifens vorliegen.

Die pH-Werte der Testlösungen bzw. der zur Herstellung des analytischen Elements verwendeten Gemische liegen zwischen den pH-Werten 3 und 10 und vorteilhafterweise zwischen den pH-Werten 4,5 und 7,5.

Zur Einstellung der pH-Werte können die in diesen pH-Bereichen wirksamen Puffer verwendet werden. Beispielhaft seien Acetatpuffer und Phosphatpuffer genannt.

Die Herstellung der erfindungsgemäßen Testlösungen ist nicht kritisch. Sie erfolgt in der Weise, daß die für eine der drei aufgeführten Verfahrensmöglichkeiten i) bis iii) erforderlichen Komponenten in den angegebenen Konzentrationen zunächst im geeigneten Puffer gelöst werden. Nach Zugabe einer unbekannten Menge der zu bestimmenden Verbindung (Wasserstoffperoxid, Oxidasesubstrat, Halogenid) kann die Anwesenheit der gesuchten Verbindung durch die Farbänderung der Testlösung nachgewiesen werden. Quantitativ kann die zu bestimmende Verbindung in der Testlösung, nach einer Wartezeit zur Farbentwicklung von 2 Minuten bis 60 Minuten, vorzugsweise von 10 Minuten bis 30 Minuten, durch spektralphotometrische Bestimmung der Zunahme der Extinktion des neu gebildeten Farbstoffs und durch Vergleich mit der Extinktion einer bekannten Konzentration der zu bestimmenden Verbindung erfaßt werden.

Zur Herstellung eines erfindungsgemäßen Teststreifens werden die entsprechenden, in geeigneten Puffern aufgelösten Komponenten in ein quellfähiges Gel eingearbeitet und auf einem Träger, wie einem Papierstreifen oder einer transparenten Folie, gleichmäßig verteilt. Im Fall der Verwendung einer transparenten Folie als Träger, z.B. einer Folie aus Celluloseacetat, Poly(ethylenterephthalat), Polycarbonaten oder Polystyrol, wird das Gel in der Art einer photographischen Schicht aufgebracht.

Als quellfähige Gele eignen sich hydrophile Materialien, wie Gelatine, Gelatinederivate, Polysaccharide, wie hydrophile Cellulosederivate, Dextran und Agarose sowie hydrophile, in Wasser lösliche, synthetische Polymere, wie Polyvinylalkohol, Polyvinylpyrrolidon, Acrylsäure- und Acrylamidpolymere, besonders aber Gelatine und Polyvinylalkohol.

Die aus dem Gel hergestellte Indikatorschicht kann gegebenenfalls noch mit organischen Vernetzern oder anorganischen Chrom- oder Aluminiumsalzen gehärtet werden. Als organische Vernetzer eignen sich insbesondere bifunktionelle Reagentien, wie Glutaraldehyd, Cyanursäurechlorid, Diisocyanate und aktivierte Ester mit mindestens zwei aktivierten Estergruppen.

Geeignete anorganische Vernetzer sind z.B. Borsäure und ihre Salze oder die Acetate, Chloride und Sulfate des Chroms und des Aluminiums.

Die als Härter der Indikatorschichten verwendeten Vernetzungsmittel werden in der Regel als 1%ige bis 15%ige, vorzugsweise 1%ige bis 5%ige Lösungen der genannten Vernetzungsmittel in Puffern - vorzugsweise solchen, die auch zur Herstellung der Gemische für die Indikatorschicht verwendet werden - oder destilliertem Wasser eingesetzt. Die Härtungszeiten betragen 5 Minuten bis 120 Minuten, vorzugsweise 10 Minuten bis 60 Minuten.

Die Indikatorschicht kann - und in dieser Anwendungsform in der Regel ungehärtet - auch Bestandteil von Teststreifen in Form der dem Stand der Technik nach bekannten mehrschichtigen, analytischen Elemente sein. Im einfachsten Fall kann die Indikatorschicht mit einer Schicht eines in Wasser unlöslichen, in organischen Lösungsmitteln jedoch löslichen Polymeren, wie Celluloseacetat oder unvernetztes Polystyrol, überzogen werden. Zu diesem Zweck werden die Teststreifen 2 bis 15 Sekunden, vorzugsweise 5 bis 10 Sekunden, in die 0,1%igen bis 5%igen, vorzugsweise 0,1%igen bis 0,5%igen, Lösungen der Polymeren in organischen Lösungsmitteln getaucht, und nach Verdunsten des organischen Lösungsmittels werden die Teststreifen zur Analyse der Analyte verwendet.

Gemäß einer weiteren Ausführungsform des erfindungsgemäßen Nachweissystems ist auf der auf dem Träger aufgebrachten Indikatorschicht, die - wie bereits ausgeführt - gegebenenfalls mit einem in Wasser unlöslichen und in organischen Lösungsmitteln löslichen Polymeren überzogen ist, weiterhin eine Ausbreitungs- bzw. Verteilerschicht vorgesehen. Solche Ausbreitungs- bzw. Verteilerschichten sind im Stand der Technik bekannt und beispielsweise in den DE-OS 28 34 713, 28 01 476, 25 32 918, 36 42 189 und der US-PS 4 050 898 beschrieben.

Bei der Ausbreitungsschicht handelt es sich um eine Schicht, die eine Flüssigkeitsprobe aufzunehmen vermag, gleichgültig, ob die Probe der Schicht direkt zugeführt wird oder von einer Schicht oder Schichten, die sich in Strömungskontakt oder Flüssigkeitskontakt mit der Ausbreitungsschicht befinden. Die Ausbreitungsschicht sorgt dafür, daß eine gleichmäßige Konzentration der zu analysierenden Probe an der Oberfläche der Ausbreitungsschicht, die der Indikatorschicht gegenüberliegt, erzeugt wird. Vorteilhafte Ausbreitungsschichten sind isotrop poröse Schichten. Unter einer isotropen Porosität ist eine Porosität in allen Richtungen einer Ausbreitungsschicht zu verstehen. Beispiele für Materialien für die Matrix der Ausbreitungsschicht sind Filterpapier, Textilmaterialien, wie Stoffe aus Kammgarn und Popelin, Glasfaserfilterpapier, Membranfilter und dreidimensionale Gitterstrukturen, welche aus polymeren Mikroperlen hergestellt wurden. Die Dicke der Ausbreitungsschicht ist variabel und hängt zum Teil von dem Volumen der zu analysierenden Probe ab. Als besonders vorteilhaft haben sich Ausbreitungsschichten mit einer Dicke von etwa 50 bis etwa 300 Mikrometer erwiesen. Je nach Beschaffenheit des zu analysierenden Systems können die Schichtstärken jedoch auch unter- oder oberhalb der angegebenen Grenzen liegen.

Des weiteren kann das erfindungsgemäße Nachweissystem auch eine Reflexionsschicht und/oder Filterschicht aufweisen, die zwischen der Indikatorschicht und der Ausbreitungs- bzw. Verteilerschicht vorgesehen wird bzw. werden. Solche Reflexions- bzw. Filterschichten sind beispielsweise in der US-PS 4 050 898 beschrieben. Diese Schichten ermöglichen es beispielsweise, Blutproben direkt, d.h. ohne vorhergehendes Zentrifugieren, spektrophotometrisch zu analysieren.

Die Anwendung der Teststreifen erfolgt in der Weise, daß auf die Indikatorschicht des Teststreifens eine definierte Menge der zu bestimmenden Verbindung aufgetragen wird oder der Teststreifen in die Probenlösung mit der zu bestimmenden Verbindung eingetaucht wird. Nach 2 Minuten bis 60 Minuten, vorzugsweise 5 Minuten bis 15 Minuten, in denen in der Indikatorschicht die Nachweisreaktion abläuft, kann die gebildete Farbe nach bekannten Methoden visuell - zur halbquantitativen Bestimmung gegebenenfalls mit einem Farbkomperator - oder photometrisch, z. B. durchlichtphotometrisch oder reflexionsphotometrisch, quantitativ ausgewertet werden.

Die erfindungsgemäßen Nachweissysteme können in der medizinischen Diagnostik zur Bestimmung von Stoffwechselmetaboliten wie Glucose, Galactose, Lactat, Cholin, Cholesterin und Harnsäure, in Laboratorien der Lebensmittelindustrie zur Bestimmung von Kohlenhydraten, Alkoholen und Aminosäuren und in der biotechnologischen Industrie zur Bestimmung der gleichen Analyte bei Meß- und Kontrollarbeiten eingesetzt werden. Weiterhin können die Testlösungen und analytischen Elemente (Teststreifen) auch, z.B. im Umweltschutz, zum Nachweis und zur Bestimmung von Halogeniden, insbesondere Bromiden und Chloriden, verwendet werden.

Bei der Herstellung der erfindungsgemäßen Nachweissysteme entfällt die Verwendung von Farbkupplern, so daß in dem erfindungsgemäßen Nachweissystem eine einfachere Kinetik der Farbstoffbildung vorliegt. Die genannten Eigenschaften des erfindungsgemäßen Nachweissystems tragen dazu bei, daß Wasserstoffperoxid, Substrate, aus denen unter der Einwirkung von Oxidasen Wasserstoffperoxid gebildet wird, und Halogenide einfach, schnell, kostengünstig, zuverlässig und spezifisch bestimmt werden können.

Die Erfindung wird durch die nachstehenden Beispiele näher erläutert.

### Beispiele

### Beispiel 1

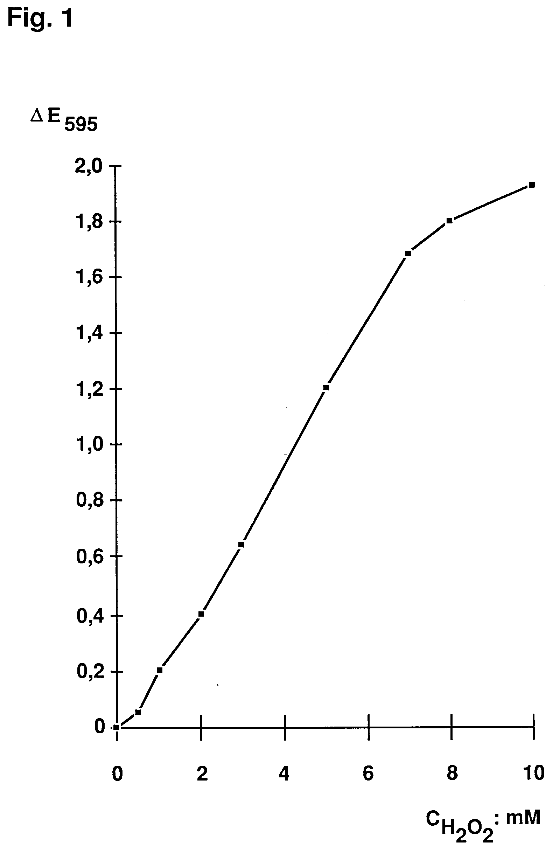

3 ml Acetatpuffer (pH-Wert 6,0; Ionenstärke 0,1), 1 ml einer Kresolpurpur-Lösung im gleichen Puffer (20 mg Kresolpurpur pro 50 ml Puffer), 1 ml einer Natriumbromid-Lösung im gleichen Puffer (10,3 g NaBr in 50 ml Puffer) und 2 Aktivitätseinheiten Halogenase aus Pseudomonas pyrrocinia werden bei Zimmertemperatur gemischt. Nach Zugabe von Wasserstoffperoxid bekannter Konzentration zu dieser Lösung entsteht aus der gelbgefärbten Lösung über grüne Zwischenstufen eine Lösung von blauvioletter Farbe, die nach 20 Minuten registriert oder auch spektralphotometrisch bei 595 nm vermessen werden kann. Als Ergebnis solcher spektralphotometrischer Messungen verschiedener bekannter Konzentrationen von Wasserstoffperoxid wurde die in Figur 1 gezeigte Kurve erhalten. Sie kann als Eichkurve zur Bestimmung unbekannter Konzentrationen an Wasserstoffperoxid verwendet werden.

### Beispiel 2

In der gleichen Weise wie in Beispiel 1 beschrieben, wird eine Lösung hergestellt, mit der Ausnahme, daß anstelle der Natriumbromid-Lösung 1 ml einer 5%igen Wasserstoffperoxidlösung zugegeben werden. Nach Zusatz von Natriumbromid zu dieser Lösung entsteht aus der gelbgefärbten Lösung über grüne Zwischenstufen eine Lösung von blauvioletter Farbe, die nach 20 Minuten registriert oder auch spektralphotometrisch bei 595 nm vermessen werden kann. Die Bestimmung unbekannter Konzentrationen an Natriumbromid ist dann analog des in Beispiel 1 beschriebenen Verfahrens möglich.

### Beispiel 3

In die im Beispiel 1 beschriebene Indikatorlösung werden Filterpapierstreifen eingetaucht. Nach dem Trocknen der Indikatorzone der Teststreifen kann Wasserstoffperoxid trockenchemisch über die Farbänderung nachgewiesen werden.

### Beispiel 4

In 10 ml Acetatpuffer (pH-Wert 6,5; Ionenstärke 0,1) werden zuerst 2 mg Kresolrot und 2 g Natriumbromid gelöst und danach 15 Aktivitätseinheiten einer L-Aminosäureoxidase und 5 Aktivitätseinheiten Halogenase aus Streptomyces aureofaciens. Dieser Lösung werden 0,5 ml auf Zimmertemperatur abgekühlter, zuvor in destilliertem Wasser zunächst vorgequollener und danach unter Erwärmen gelöster Polyvinylalkohol (1 g Polyvinylalkohol pro 50 ml destilliertes Wasser) unter Rühren zugesetzt. Nach der Durchmischung werden Filterpapierstreifen mit dieser Lösung getränkt, und nach dem Trocknen werden die Teststreifen zum Nachweis von L-Aminosäuren verwendet. Die durch die enzymatisch katalysierte Reaktion entstehende Farbe wechselt von gelb nach purpur.

Die Auswaschbeständigkeit einzelner Komponenten sowie die Feuchtigkeitsempfindlichkeit dieser Teststreifen kann durch Härtung der Indikatorschicht durch 5-minütiges Eintauchen der Teststreifen in eine 3%ige Borsäure-Lösung verbessert bzw. verringert werden. Die so nachbehandelten Teststreifen werden nach dem Abspülen überschüssiger Borsäure-Lösung mit destilliertem Wasser bei 40°C getrocknet.

### Beispiel 5

2 mg Phenolrot und 2 g Natriumbromid werden in 10 ml Acetatpuffer mit einem pH-Wert von 5,5 und einer Ionenstärke von 1,0 gelöst. In diese Lösung werden bei 40°C 400 mg im gleichen Acetatpuffer vorgequollene Gelatine, 1 ml Glycerin und 0,4 ml Tween 100 (als Weichmacher und Netzmittel) unter Rühren eingetragen. Nach dem Abkühlen auf Zimmertemperatur werden ebenfalls unter Rühren 30 Aktivitätseinheiten Glucoseoxidase und 20 Aktivitätseinheiten Halogenase aus Pseudomonas pyrrocinia zu der Lösung gegeben. Mit dieser Lösung werden Streifen einer transparenten Polyesterfolie beschichtet. Zur Verfestigung der Indikatorschicht werden die Teststreifen bei 40°C getrocknet und unter trockenen Bedingungen gelagert. Zur Glucosebestimmung wird auf die Teststreifen eine glucosehaltige Flüssigkeit getropft, entweder als reine Glucoselösung mit bekanntem Glucosegehalt (z.B. zur Erstellung eines Eichstandards (Eichteststreifen, Farbkomperator)) oder im Stoffgemisch (z.B. in Blut, Serum, Urin, Fermentationslösungen oder Nahrungsmittelproben) mit anderen Substanzen. Nach 10 Minuten werden überschüssige Probenlösungen mit destilliertem Wasser abgespült. 30 Minuten, 24 Stunden und 2 Wochen nach dem Abspülvorgang wird die Extinktion der blauvioletten Lösung bei 595 nm gemessen. In den genannten Zeiten verändert sich die Extinktion praktisch nicht. Diese Extinktionswerte können durch Vergleich mit Extinktionswerten der Eichstandards (Eichung mit Eichteststreifen) zur quantitativen Bestimmung unbekannter Glucosekonzentrationen herangezogen werden. Durch Vergleich mit den Farben eines Farbkomperators können unbekannte Glucosekonzentrationen halbquantitativ durch visuelle Betrachtung bestimmt werden. Bei diesen Analysen wurde eine Abhängigkeit der Extinktion bzw. Farbentwicklung von der Glucosekonzentration bis etwa 30 mMol gefunden.

Zur Verbesserung der Wischfestigkeit und mechanischen Stabilität sowie zur Verringerung der Feuchtigkeitsempfindlichkeit kann die Indikatorschicht gehärtet werden. Dazu wird die Indikatorschicht der Teststreifen 5 Minuten entweder in eine 2,5%ige Glutaraldehyd-Lösung im obigen Acetatpuffer oder in eine 1%ige Chrom (III)-chlorid-Lösung (Chrom(III)-chlorid als Hexahydrat eingesetzt) im obigen Acetatpuffer getaucht. Nach dem Abspülen des überschüssigen Vernetzungsmittels mit destilliertem Wasser werden die Teststreifen bei 40°C getrocknet.

Wischfestigkeit, mechanische Stabilität und Feuchtigkeitsempfindlichkeit können auch durch Überzug der Indikatorschicht mit einem transparenten, in Wasser unlöslichen und in organischen Lösungsmitteln löslichen Polymeren verbessert werden. Zu diesem Zweck wird die Indikatorschicht der Teststreifen z.B. in eine 0,4%ige Lösung von Celluloseacetat in Chloroform oder eine 0,5%ige Lösung von unvernetztem Polystyrol in Tetrahydrofuran 2 Sekunden eingetaucht. Nach dem Abdampfen des organischen Lösungsmittels bei Zimmertemperatur können die Teststreifen zur Bestimmung der Glucose verwendet werden.

## Patentansprüche

1. Nachweissystem zur qualitativen und quantitativen Bestimmung von Wasserstoffperoxid, Substraten, aus denen unter der Einwirkung von Oxidasen Wasserstoffperoxid gebildet wird, und Halogeniden, dadurch **gekennzeichnet,** daß
i) zur Bestimmung von Wasserstoffperoxid das Nachweissystem
(a) mindestens einen halogenierbaren Farbstoff,
(b) mindestens ein halogenierendes Enzym, und
(c) mindestens ein anorganisches Halogenid
enthält; oder
ii) zur Bestimmung von Substraten, aus denen unter der Einwirkung von Oxidasen Wasserstoffperoxid gebildet wird, das Nachweissystem
(a) mindestens einen halogenierbaren Farbstoff,
(b) mindestens ein halogenierendes Enzym,
(c) mindestens ein anorganisches Halogenid, und
(d) mindestens eine Oxidase
enthält; oder
iii) zur Bestimmung von Halogenid das Nachweissystem
(a) mindestens einen halogenierbaren Farbstoff,
(b) mindestens ein halogenierendes Enzym, und
(e) Wasserstoffperoxid
enthält.

2. Nachweissystem nach Anspruch 1, dadurch **gekennzeichnet,** daß der halogenierbare Farbstoff (a) ein Triarylmethanfarbstoff ist.

3. Nachweissystem nach Anspruch 2, dadurch **gekennzeichnet,** daß der Triarylmethanfarbstoff Phenolrot, Chlorphenolrot, Kresolrot, Kresolpurpur, Thymolblau, Xylenolblau oder Xylenolorange ist.

4. Nachweissystem nach einem der Ansprüche 1 bis 3, dadurch **gekennzeichnet,** daß das halogenierende Enzym (b) eine Halogenase aus Pseudomonas pyrrocinia oder Streptomyces aureofaciens ist.

5. Nachweissystem nach einem der Ansprüche 1 bis 4, dadurch **gekennzeichnet,** daß das anorganische Halogenid (c) ein Alkali- oder Erdalkalihalogenid ist.

6. Nachweissystem nach Anspruch 5, dadurch **gekennzeichnet,** daß das anorganische Halogenid (c) ein Alkali- oder Erdalkalibromid ist.

7. Nachweissystem nach einem der Ansprüche 1 bis 6, dadurch **gekennzeichnet,** daß die Oxidase (d) Glucoseoxidase, Galactoseoxidase, Lactatoxidase, Cholinoxidase, Cholesterinoxidase, Uricase, L- bzw. D-Aminosäureoxidase, Alkoholoxidase, Xanthinoxidase oder Monoaminoxidase ist.

8. Nachweissystem nach einem der Ansprüche 1 bis 7, dadurch **gekennzeichnet,** daß es in Form einer die Komponenten (a), (b) und (c) oder die Komponenten (a), (b), (c) und (d) oder die Komponenten (a), (b) und (e) enthaltenden Lösung vorliegt.

9. Nachweissystem nach einem der Ansprüche 1 bis 7, dadurch **gekennzeichnet,** daß die Komponenten (a), (b) und (c) oder die Komponenten (a), (b), (c) und (d) in ein Gel eingearbeitet sind, und das Gel auf einem Träger als Indikatorschicht aufgebracht ist.

10. Nachweissystem nach Anspruch 9, dadurch **gekennzeichnet,** daß der Träger eine transparente Folie ist.

11. Nachweissystem nach Anspruch 9 oder 10, dadurch **gekennzeichnet,** daß das Gel weitere Zusätze, ausgewählt aus Weichmachern, Netzmitteln und/oder Puffersubstanzen, eingearbeitet enthält.

12. Nachweissystem nach Anspruch 11, dadurch **gekennzeichnet,** daß die aus dem Gel hergestellte Indikatorschicht mit einem organischen oder anorganischen Vernetzer gehärtet worden ist.

13. Nachweissystem nach Anspruch 12, dadurch **gekennzeichnet,** daß die aus dem Gel hergestellte Indikatorschicht mit einem organischen Vernetzer, ausgewählt aus Glutaraldehyd, Cyanursäurechlorid, Diisocyanaten und aktivierten Estern mit mindestens zwei aktivierten Estergruppen, oder mit einem anorganischen Vernetzer, ausgewählt aus Borsäure, deren Salzen oder Acetaten, Chloriden und Sulfaten des Chroms und des Aluminiums, vernetzt worden ist.

14. Nachweissystem nach einem der Ansprüche 9 bis 11, dadurch **gekennzeichnet,** daß die auf den Träger aufgebrachte Indikatorschicht mit einem in Wasser unlöslichen und in organischen Lösungsmitteln löslichen Polymeren überzogen ist.

15. Nachweissystem nach Anspruch 14, dadurch **gekennzeichnet,** daß das Polymere Celluloseacetat oder unvernetztes Polystyrol ist.

16. Nachweissystem nach einem der Ansprüche 14 oder 15, dadurch **gekennzeichnet,** daß auf der auf dem Träger aufgebrachten Indikatorschicht, die gegebenenfalls mit einem in Wasser unlöslichen und in organischen Lösungsmitteln löslichen Polymeren überzogen ist, weiterhin eine Ausbreitungs- bzw. Verteilerschicht vorgesehen ist.

17. Nachweissystem nach Anspruch 16, dadurch **gekennzeichnet,** daß zwischen der Indikatorschicht und der Ausbreitungs- bzw. Verteilerschicht eine Reflexionsschicht und/oder Filterschicht vorgesehen sind.

18. Verfahren zur qualitativen und quantitativen Bestimmung von Wasserstoffperoxid, Substraten, aus denen unter der Einwirkung von Oxidasen Wasserstoffperoxid gebildet wird, und Halogeniden, dadurch **gekennzeichnet,** daß man einen Analyten mit einem Nachweissystem nach einem der Ansprüche 1 bis 17 in Kontakt bringt und die Farbänderung qualitativ oder quantitativ bestimmt.

19. Verfahren nach Anspruch 18, dadurch **gekennzeichnet,** daß man als Substrate, aus denen unter der Einwirkung von Oxidasen Wasserstoffperoxid gebildet wird, Glucose, Galactose, Lactose, Raffinose, Stachyose, Melibiose, Lactat, Cholin, Cholesterin, Harnsäure, L- oder D-Aminosäuren, Methanol, Ethanol, Xanthin und/oder Amine verwendet.
